# EUROPEAN PATENT APPLICATION

(11) **EP 4 843 478 A1**
(43) Date of publication of application: **25.11.2026**
(21) Application number: 25177376.8
(22) Date of filing: 19.05.2025
(51) Int. Cl.: A61B 5/00, A61B 5/318

(54) **PATIENT MONITORING DEVICE, SYSTEM AND METHOD AND MEASUREMENT ACCESSORY**

(71) Applicant: Koninklijke Philips N.V., 5656 AE Eindhoven (NL)
(72) Inventor: NEUMANN, Rolf, 5656 AG Eindhoven (NL)
(74) Representative: Philips Intellectual Property & Standards

(57) **Abstract**

The present invention relates to a patient monitoring device, system and method. The patient monitoring device (10) comprises a plurality of signal interfaces (11-15) configured to obtain a plurality of electrode signals (41-45) acquired by electrodes (21-25) placed at different positions at a patient's body; electrocardiographic (ECG) signal processing circuitry (16) configured to apply ECG signal processing to a first set of electrode signals obtained at a first set of signal interfaces of the plurality of signal interfaces to obtain ECG information; and electrophysiological (EP) signal processing circuitry (17) configured to apply EP signal processing different from the ECG signal processing to a second set of electrode signals obtained at a second set of signal interfaces of the plurality of signal interfaces to obtain EP information. In a first operation mode, the ECG signal processing circuitry (16) is configured to apply ECG signal processing to the first set of electrode signals and the EP signal processing circuitry (17) is configured to apply EP signal processing to the second set of electrode signals, and, in a second operation mode, the ECG signal processing circuitry (16) is configured to apply ECG signal processing to the first and second sets of electrode signals.

## Description

### FIELD OF THE INVENTION

The present invention relates to a patient monitoring device, a patient monitoring system and a patient monitoring method. The present invention relates further to measurement accessories, in particular for use in a such a patient monitoring system.

### BACKGROUND OF THE INVENTION

Electromyography (EMG) is a non-invasive diagnostic measurement to detect and measure the electrical signals generated by muscle fibres during contraction and relaxation. It involves the use of electrodes that are placed on the skin close to the muscle(s) that shall be analysed. It is a well-known approach to use EMG signals especially also to measure the corresponding muscle activity driving the respiration to not only derive the respiration rate, which can be determined by multiple other approaches, but also to get a qualitative or even quantitative measure of the neural respiratory drive and respiration effort.

The application of EMG based respiratory effort measurement is already available as medical device functionality. One application is to use the Surface EMG-based Estimation of Breathing Effort for Neurally Adjusted Ventilation Control, as e.g. described in Eike Petersen, Jan Graßhoff, Marcus Eger, Philipp Rostalski, Surface EMG-based Estimation of Breathing Effort for Neurally Adjusted Ventilation, IFAC-PapersOnLine, Volume 53, Issue 2, 2020, Pages 16323-16328, ISSN 2405-8963, https://doi.org/10.1016/j.ifacol.2020.12.654. Another application is to measure the EMG activity of the diaphragm of a newborn or to measure the diaphragm muscle activity to improve the ventilation of a patient. It was found that an EMG specific electrode placement is needed, and the electrode placement should therefore be optimized for the EMG measurement to make sure that respiration information would be available consistently.

Extracting respiration related EMG signal components from standard ECG signal recordings have previously been described, e.g. Forbes, A. Dean, and Eric D. Helfenbein. "Apparatus and method for determining respiratory effort from muscle tremor information in ECG signals," U.S. Patent 5,913,308, issued June 22, 1999. The performance of this approach is limited by the artifacts from other, non-respiratory muscle activities that are superimposed to the signals obtained with standard ECG lead placement, as e.g. described in Eric Helfenbein, Reza Firoozabadi, Simon Chien, Eric Carlson, Saeed Babaeizadeh, Development of three methods for extracting respiration from the surface ECG: A review, Journal of Electrocardiology, Volume 47, Issue 6, 2014, Pages 819-825, ISSN 0022-0736, https://doi.org/10.1016/j.jelectrocard.2014.07.020.

To reduce the impact of non-respiratory muscle activity a specialized EMG electrode placement that is different to the standard ECG electrode placement is important. It has been demonstrated that a parasternal electrode position maximizes the contribution of 2nd ICS parasternal muscle to the inspiratory EMG signal and minimizes the non-respiratory muscle activity of other muscles, as e.g. described in Nicholas Hart, John Moxham, Fiammetta Fedele, "PATIENT MONITORING METHOD AND MONITORING DEVICE" U.S. Patent 10,617,307, issued April 14, 2020.

Overall, there is clinical interest, but besides a few exceptions, the use of respiratory EMG is still mostly experimental and limited in clinical practice. One aspect is limited knowledge on its validity and clinical relevance, but a widespread implementation is also hindered by practical challenges and pitfalls for acquisition.

### SUMMARY OF THE INVENTION

It is an object of the present invention to overcome workflow hurdles in clinical use and enable that electrophysiological measurements like EMG can become a standard measurement for patient monitoring.

In a first aspect of the present invention a patient monitoring device is presented comprising:
- a plurality of signal interfaces configured to obtain a plurality of electrode signals acquired by electrodes placed at different positions at a patient's body;
- electrocardiographic (ECG) signal processing circuitry configured to apply ECG signal processing to a first set of electrode signals obtained at a first set of signal interfaces of the plurality of signal interfaces to obtain ECG information; and
- electrophysiological (EP) signal processing circuitry configured to apply EP signal processing different from the ECG signal processing to a second set of electrode signals obtained at a second set of signal interfaces of the plurality of signal interfaces to obtain EP information,
   wherein,
- in a first operation mode, the ECG signal processing circuitry is configured to apply ECG signal processing to the first set of electrode signals and the EP signal processing circuitry is configured to apply EP signal processing to the second set of electrode signals, and,
- in a second operation mode, the ECG signal processing circuitry is configured to apply ECG signal processing to the first and second sets of electrode signals.

In a further aspect of the present invention a measurement accessory, in particular measurement cable or lead-set, is presented that is configured to acquire and/or transport an electrode signal acquired by an electrode of a plurality of electrodes configured to be placed at different positions at a patient's body and to acquire electrode signals, and to convey its signal propagation properties to a patient monitoring device as disclosed herein.

In a further aspect of the present invention a measurement accessory, in particular measurement cable or lead-set, is presented that is configured to acquire and/or transport an electrode signal acquired by an electrode of a plurality of electrodes configured to be placed at different positions at a patient's body and to acquire electrode signals, and to convey its intended operation mode or to convey the intended purpose of individual leads to a patient monitoring device as disclosed herein.

In a further aspect of the present invention a measurement accessory, in particular measurement cable or lead-set, is presented that is configured to acquire and/or transport an electrode signal acquired by an electrode of a plurality of electrodes configured to be placed at different positions at a patient's body and to acquire electrode signals, and to convey the intended positioning of the leads on the body or to convey the intended EP signal processing function to a patient monitoring device as disclosed herein.

In a further aspect of the present invention a patient monitoring system is presented comprising:
- measurement accessory used for acquiring and/or transporting the electrode signals acquired by a plurality of electrodes configured to be placed at different positions at a patient's body and to acquire electrode signals; and
- a patient monitoring device as disclosed herein.

In yet further aspects of the present invention, there are provided a corresponding patient monitoring method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to combine the signal acquisition for EMG monitoring (or, more generally, EP monitoring) with the signal acquisition for ECG monitoring into one measurement module (the patient monitoring device) and allow simultaneous signal acquisition while the electrodes can be optimally placed for both measurements, for instance for an EMG-based respiratory monitoring function as well as for the ECG monitoring function. The one measurement module may share common functions for both, the EP signals and the ECG signals (e.g. signal acquisition hardware, patient isolation means, or common reference electrode) and comprises separate processing circuitry (e.g. dedicated processing units, processors, or programmed processing means, etc.) for separately processing the acquired EP and ECG signals. Further, there are different operation modes depending on which kind of signals are acquired by the connected electrodes.

The present invention is thus related to patient monitoring equipment and its use to flexibly obtain and process EP and/or ECG signals using the same equipment, preferably including the same electrodes, which are all either optimally placed for ECG monitoring or which are partly optimally placed for ECG signal acquisition and partly (e.g. a single electrode pair) optimally places for EP signal acquisition (e.g. EMG signal acquisition). Thus, for EP signal acquisition and processing, no separate equipment is required which may overcome workflow hurdles in clinical use and may enable that EP measurements like EMG can become a standard measurement for patient monitoring.

While respiratory effort determination is a key application, the present invention is not limited to EMG based respiratory measurement (as one embodiment of EP measurement) and to only measure the activity of the respiration muscles, but it can be applied to any EMG based muscle activity measurement or other EP signals. Some of the benefits of the present invention may be most relevant for EMG applications, which are related to muscles that are in relative proximity to the heart or any EMG signals where a significant cardiac crosstalk is observed. Nevertheless, whenever reference is made to "EMG" herein, it shall generally be understood in a broad sense as a reference to "EP", unless otherwise specified. For instance, a reference to EMG measurements shall generally be understood as a reference to EP measurements, which may include but are not limited to EMG measurements.

In an embodiment the EP signal processing circuitry is configured to use, in the first operation mode, one or more of the first set of electrode signals and/or a processing result of the simultaneous processing of the ECG signal processing circuitry of the first set of electrode signals in the processing of the second set of electrode signals. This may improve the accuracy and reliability of the EP information resulting from the processing of the second set of electrode signals.

For instance, as provided in an exemplary embodiment, an optimized removal of cardiac crosstalk in the EP signal (in particular in EMG signals) may be provided, for which purpose the EP signal processing circuitry may be configured to use, in the first operation mode, one or more of the first set of electrode signals and/or a processing result of the simultaneous processing of the ECG signal processing circuitry of the first set of electrode signals to reduce or remove cardiac crosstalk in the second set of electrode signals, in particular by use of one or more of:
- timing and/or amplitude and/or shape of a QRS complex in one or more of the first set of electrode signals, and
- presence and/or removal of pacer spikes in one or more of the first set of electrode signals.

Further, as provided in another exemplary embodiment, an optimized handling of external noise in the EP signal (in particular in EMG signals) may be provided, for which purpose the EP signal processing circuitry may configured to use, in the first operation mode, one or more of the first set of electrode signals and/or a processing result of the simultaneous processing of the ECG signal processing circuitry of the first set of electrode signals to reduce or remove the impact of external noise components in the second set of electrode signals, in particular by use of one or more of:
- common mode noise visible in one or more electrode signals of the first set,
- instances of time in which external noise above a threshold is present one or more electrode signals of the first set,
- a virtual reference electrode signal for the electrode signal of the second set from at least one electrode signal of the first set.

The ECG signal processing circuitry and/or the EP signal processing circuitry may be configured in an embodiment to identify if an electrode signal obtained at a signal interface is an ECG signal or another EP signal and to switch the ECG signal processing circuitry into the first or second operation mode depending on the result of said identification, in particular to switch the ECG signal processing circuitry into the second operation mode if no EP signal different from an ECG signal has been identified at any signal interface. In one embodiment the user may generally connect the electrodes to any one of the signal interfaces, i.e., there are no signal interfaces to which only electrodes for ECG signal acquisition can be connected and no signal interfaces to which only electrodes for EP signal acquisition can be connected, but the signal interfaces are configured for universal use. The signal processing in the patient monitoring device will automatically detect if a connected electrode provides an ECG signal or an EP (e.g. EMG) signal, which mainly depends on the location of the signal acquisition, i.e., the location where the electrode is placed at the patient's body. This embodiment hence provides improved usability features to identify the EP signals from the correct electrodes and distinguish them clearly from the ECG signals.

According to another embodiment, the EP signal processing circuitry is configured to reference the second set of electrode signals to one of:
- a reference signal among the first set of electrode signals,
- a non-reference signal among the first set of electrode signals,
- another electrode signal of the second set of electrode signals, and
- a combination of multiple electrode signals obtained at any signal interface.

The default option is to use a reference signal that is already applied to the patient and that is optimized, e.g. to reduce the common-mode-voltage (Right-Leg Drive). The non-reference signal may be beneficial when such a signal provides a better reference to suppress cardiac overlay and the burden to place a separate EP reference electrode at the thorax shall be avoided. The other electrode signal may be beneficial if the EP signals are obtained at a body location different from the thorax, e.g. targeting the diaphragm or if the EP signal quality is so much improved that a separate EP reference electrode at the thorax can be justified. The combination of multiple electrode signals combines any of the electrode signals to a "virtual" reference.

The patient monitoring device may optionally further comprise a control unit configured to switch the ECG signal processing circuitry and/or the EP signal processing between the first operation mode and the second operation mode based on user input and/or based on accessory information conveyed by the measurement accessory used for acquiring and/or transporting the electrode signals and/or based on an evaluation of one or more parameters of obtained electrode signals. This improves user handling and operation since the switching between the operation modes either happens automatically or based on simple user input, e.g., an input identifying the specific type of accessory that is currently used (e.g. selected from a list or entering an alpha-numeric code) or an input indicating that currently an electrode has been placed at a position for EP signal acquisition or not. Measurement accessories, such as trunk cables, lead-sets, electrodes, etc. may carry information, e.g. as accessory identifier or other unique information (e.g. a QR code or barcode or alpha-numeric code or color code or data storage means) that allows the patient monitoring device to acquire this information and recognize which kind of signals shall be acquired and, hence, which operation mode shall be used. For instance, special trunk cables and/or lead-sets may be used to identify the dual use, i.e., the simultaneous use for ECG and EP measurements.

According to another embodiment, the EP signal processing is configured to apply or adapt EP signal processing based on user input and/or based on accessory information conveyed by the measurement accessories used for acquiring and/or transporting the electrode signals and/or based on an evaluation of one or more parameters of obtained electrode signals.

Furthermore, in an embodiment, the patient monitoring device may optionally further comprise a user interface configured to notify the user of the operation mode and/or any mismatch regarding the actual operation mode based on accessory information conveyed by the measurement accessory used for acquiring and/or transporting the electrode signals and/or based on an evaluation of one or more parameters of obtained electrode signals. The user interface may generally be any interface that can provide information to the user, such as a display, loudspeaker, monitor, touchscreen, etc.

According to another embodiment, the patient monitoring device may further comprise an annotation unit configured to annotate the obtained ECG information and/or the obtained EP information based on one or more of the operation mode, accessory information conveyed by the measurement accessory used for acquiring and/or transporting the electrode signals, and an evaluation of one or more parameters of obtained electrode signals. In this way, the respective information can be automatically annotated which reduces workload of the user but ensures that the information is correctly annotated as EP or ECG information.

In another embodiment the first set of signal interfaces comprises at least three signal interfaces and the second set of signal interfaces comprises at least one signal interface. Thus, at least a 3-lead ECG measurement can be made in addition to an EP signal measurement. While there may be dedicated signal interfaces (e.g. connectors such as plugs or sockets for respective cables), in another embodiment, the first and second sets of signal interfaces are no predetermined signal interfaces, i.e., the signal information can be flexibly used for both types of measurements.

The ECG signal processing circuitry is preferably configured for ECG based monitoring and/or to obtain ECG based cardiac information and/or for impedance respiration monitoring and/or to obtain impedance respiratory information. The EP signal processing circuitry is preferably configured to apply electromyographic (EMG) signal processing, in particular for EMG based respiratory monitoring and/or to obtain one or more of respiratory phase (e.g. for optimizing ventilation cycles), respiratory trigger signal (e.g. for optimizing ventilation cycles), respiration rate, breathing effort, and breathing wave.

According to another embodiment of the patient monitoring device, in a third operation mode, the ECG signal processing circuitry may be configured to suspend ECG signal output, and the EP signal processing circuitry may be configured to apply EP signal processing to the second set of electrode signals. Hence, in this third operation mode, only EP signal processing is carried out, but no ECG signal processing.

As mentioned above, another aspect of the present invention relates to measurement accessory, in particular measurement cable or lead-set, is presented that is configured to acquire and/or transport an electrode signal acquired by an electrode of a plurality of electrodes configured to be placed at different positions at a patient's body and to acquire electrode signals, and to convey one or more of its signal propagation properties, its intended operation mode, its intended purpose, an intended electrode position on the patient's body, an intended patient population, and an intended EP signal processing function to a patient monitoring device as disclosed herein.

The signal propagation properties may include one or more of length of signal wire, length of cable, capacity of signal wire to shield (expressed as capacity per length), capacity of signal wire to other signal wires (expressed as capacity per length), total capacity of signal wire to shield, total capacity of signal wire to other signal wires, inductance of signal wire (expressed as inductance per length), total inductance of signal wire, filter characteristic of signal wire when connected to a known impedance of the signal interface, electrosurgical unit (ESU) related protection means yes/no, impedance of ESU related protection means, signal amplitude protection means yes/no, characteristics of signal amplitude protection means, wire class identifier with properties known by the patient monitoring device, cable class identifier with properties known by the patient monitoring device.

Digital or analog means may be used to transfer the accessory's signal propagation properties to the patient monitoring device, such as an NFC chip, memory, micro controller, resistors, capacitors, inductors, light reflection, light absorption, bar codes, QR codes, alpha-numeric codes, etc. The patient monitoring device may then use these signal propagation properties to optimize the ECG signal processing and/or the EP signal processing. These means may either be integrated into the connector or "permanently" attached to the accessory.

The measurement cable or lead-set may be configured to be used for acquiring and/or transporting electrode signals from different positions at a patient's body to perform with a single cable or lead-set simultaneous ECG and EP (EMG) monitoring and to convey its intended operation mode or to convey the intended purpose of the individual leads for either ECG signal processing, EP signal processing, as ECG reference signal or as EP reference signal to a patient monitoring device as disclosed herein. The conveyed information may include one or more of intended accessory operating mode (e.g. ECG signal processing and/or EP signal processing), intended purpose of one or more individual leads transporting an electrode signal (e.g. lead identifier indicating the intended position of the corresponding electrode on the patient's body, ECG lead yes/no, ECG lead identifier indicating the intended position of the corresponding ECG electrode on the patient's body, impedance respiration lead yes/no, impedance respiration alternative lead in case of signal disturbance on another impedance respiration lead, EP lead yes/no, EP lead identifier indicating the intended position of the EP electrode on the patient's body, reference lead for ECG, reference lead for impedance respiration, reference lead for EP).

Digital or analog means may be used to transfer the accessory's operation mode or individual lead purpose to the patient monitoring device, such as an NFC chip, memory, micro controller, resistors, capacitors, inductors, light reflection, light absorption, bar codes, QR codes, alpha-numeric codes, etc. The patient monitoring device may then use the information about the accessory's operation mode or individual lead purpose to automatically select the proper operating mode and/or to automatically assign the signals received at the signal interfaces for the processing or to inform the user in case a mismatch is detected between the information conveyed by the accessory and a setting selected in the patient monitoring device.

The measurement cable or lead-set may be configured to be used for acquiring and/or transporting electrode signals from different positions at a patient's body to perform EP (e.g. EMG) monitoring and to convey the intended function/positioning of the leads on the body or to convey the intended EP signal processing function to a patient monitoring device. The conveyed information may include one or more of EP processing function (e.g. EMG, EEG, other EP function, parasternal respiratory EMG, diaphragm respiratory EMG, adult EMG, pediatric EMG, neonatal EMG), EP lead identifier indicating the intended function of the corresponding EP electrode (e.g. parasternal respiratory EMG lead 1 or 2, diaphragm respiratory EMG lead 1 or 2, reference lead for EP, adult chest lead A or B, adult abdomen lead A or B, pediatric chest lead A or B, pediatric abdomen lead A or B, neonatal chest lead A or B, neonatal abdomen lead A or B), lead identifier indicating the intended position of the corresponding electrode on the patient's body (e.g. parasternal left or right, diaphragm left or right, leg left or right, arm left or right, abdomen, chest, head), intended patient population for the accessory (e.g. adult, pediatric, neonatal).

Digital or analog means may be used to transfer the accessory's EP signal processing function or the individual EP lead purpose to the patient monitoring device, such as an NFC chip, memory, micro controller, resistors, capacitors, inductors, light reflection, light absorption, bar codes, QR codes, alpha-numeric codes, etc. The patient monitoring device may then use the conveyed information e.g. to automatically select the EP signal processing function between a parasternal EMG electrode placement versus a diaphragm EMG electrode placement or optimize the EP processing function for adult patients versus neonatal patients. Alternative to an automatic selection of the processing, the conveyed accessory information may be used by the patient monitoring device to perform a check versus a setting in the patient monitoring device and to inform the user in case of a mismatch.

### BRIEF DESCRIPTION OF THE DRAWINGS

These and other aspects of the invention will be apparent from and elucidated with reference to the embodiment(s) described hereinafter. In the following drawings:
Fig. 1A shows a schematic diagram of a first embodiment of a patient monitoring system according to the present invention.
Fig. 1B shows a schematic diagram of a second embodiment of a patient monitoring system according to the present invention.
Fig. 2 shows a schematic diagram of an embodiment of a patient monitoring device according to the present invention.
Fig. 3 shows a schematic diagram of another embodiment of a patient monitoring device according to the present invention.

### DETAILED DESCRIPTION OF EMBODIMENTS

Current approaches to measure respiratory effort (and/or respiratory rate and/or respiratory phase) with EMG have a clinical workflow and/or cost burden because they either:
a) use a dedicate EMG device to capture the EMG signals with electrodes placed at the preferred body position (e.g. the diaphragm, intercostal muscles or accessory muscles) to measure the respiration activity, or
b) use a standard ECG measurement device and the signals obtained from standard ECG electrode placements, or
c) use a standard ECG measurement device but optimize the signals for the EMG use case to measure the respiration activity by placing the electrodes to best capture the respiratory muscle activity, which is different to the electrode placement that is needed for standard ECG interpretation.

The disadvantages of these known approaches include that they require additional equipment and additional trunk cables between the patient monitor and the patient (e.g. to reach the patient in the bed), compromise on the EMG signal quality wherein specific EMG monitoring of the respiration relevant muscles is not possible, and cannot provide the standardized ECG leads and therefore might be misinterpreted by caregivers and/or cause ECG analysing algorithms to draw false conclusions.

In acute care environments an ECG measurement is often indicated and therefore already available. A standardized ECG electrode placement is needed to obtain standardizes ECG leads and avoid misinterpretation. Depending on the monitoring need 3-, 5-, 6- or even 12-lead ECG signals are monitored, whereas the 12-lead ECG acquisition is dominantly done as a snapshot for diagnostic purposes, and only for a few cases in form of a continuous monitoring. Therefore, in most cases only 3-, 5- or 6- lead ECG monitoring is used in critical care settings. The reason to limit the monitoring leads in most cases to less than 12 leads is typically not per se a technical limitation of the ECG measurement devices, but a conscious decision due to clinical workflow and patient comfort considerations in order to limit the number of electrodes to the minimum necessary.

While in a neonatal application the heart rate information might be sufficient in most cases, the specific ECG morphology is important and evaluated for diagnostic purposes in other patient populations. Therefore an electrode placement deviating from the standard ECG placement in order to optimize the EMG measurement is in general not a viable option.

Others prefer a parasternal electrode placement over the diaphragm, simplifying the measurement of neural respiratory drive index (parasternal electromyogram to measure respiratory rate and respiratory effort). The parasternal electrode placement for EMG deviates from the standard ECG electrode placement as well.

The present invention makes use of the recognition that a standard ECG measurement device is generally available for acute care patients and that technically more signal channels are available with common ECG measurement devices than are typically used for continuous ECG monitoring. The functionality of a standard ECG measurement device can therefore be extended by using "free" signal channels to capture additional signals for EMG analysis (or, more generally, for EP analysis). The additional electrodes for the EMG signal capturing can be placed independently from the standard ECG electrode placement, and therefore the placement of the EMG related electrodes can be optimized for the measurement of the respiration activity and per preference of the caregiver, like e.g. diaphragm or parasternal.

The processing of the EMG signals is a general challenge as the signals captured with the skin electrodes always contain not only the EMG signal components, but also signals coming from the heart activity, which are typically an order of magnitude larger than the EMG signals. There are many publications on how to best remove the heart signal components from the signal to extract the EMG signals. Embodiments of the present invention present solutions on how to improve the signal quality and the removal of the heart signal components, also called cardiac crosstalk. The combination of the ECG and EMG (or EP) measurements in one measurement module has the additional advantage that the EMG signals are captured in a highly synchronized manner together and in relationship with the standard ECG signals, and therefore the removal of the cardiac crosstalk can be done in a more controlled and precise manner than it would be possible with a separate EMG measurement device.

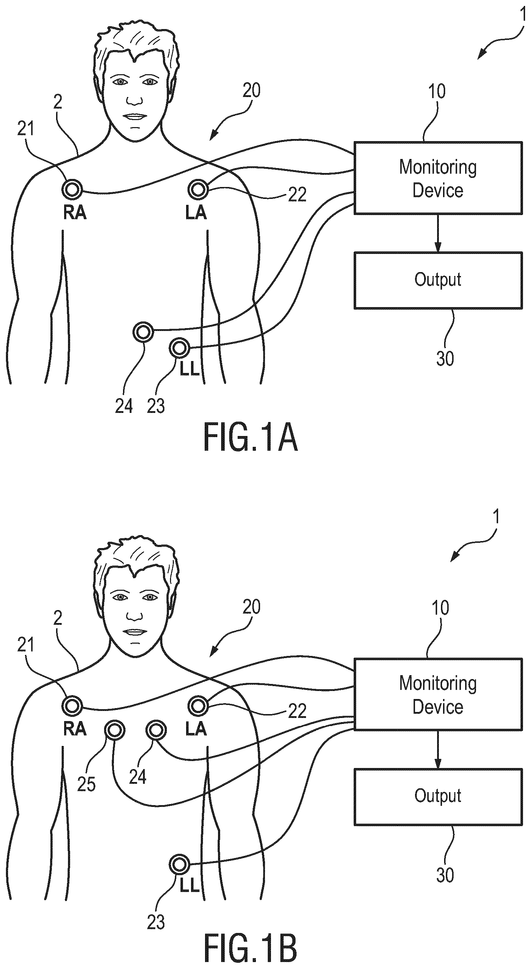

Fig. 1A shows a schematic diagram of a first embodiment of a patient monitoring system 1 according to the present invention. The system 1 comprises a patient monitoring device 10 according to the present invention and measurement accessories 20 used for acquiring and/or transporting electrode signals.

Generally, the patient monitoring device 10 may be implemented by respective units or circuitry, e.g. a processor, processing circuitry, a computer, dedicated hardware, etc., that carries out the functions of the patient monitoring device 10. Alternatively, a common unit or circuitry, e.g. a common processor or computer, may implement the various functions of the device, or separate units or elements may be used that together represent the circuitry. In an exemplary implementation, a programmed processor may represent the device 10, which may execute a computer program that may be stored in a memory that is accessed by the processor. In an embodiment, the patient monitoring device 10 may be configured as conventional patient monitor or as a measurement device that is configured to acquire and process the electrode signals, including ECG signals and EP signals.

The measurement accessories 20 include a plurality (in this example four) of electrodes 21-24 that are configured to be placed at different positions at the body of the patient 2 and to acquire electrode signals. In the exemplary use illustrated in Fig. 1, three electrodes 21-23 form an ECG 3-lead and are placed at the typical positions RA, LA and LL for acquiring ECG signals. The fourth electrode 24 is placed in the area of the diaphragm in order to acquire an EMG signal for use in determining respiratory information, such as respiration rate, respiration effort, etc.

The patient monitoring device 10 thus combines the signal acquisition for EP (e.g. EMG) monitoring with the signal acquisition for ECG monitoring into one device and allows simultaneous signal acquisition. The electrodes 21-24 may include further electrodes for acquiring ECG signals, e.g., to form a 5-lead, 10-lead or 12-lead, and/or further electrode(s) for acquiring further EP signal(s)) They can be independently placed at the optimal position for the respective signal acquisition.

Fig. 1B shows a schematic diagram of a second embodiment of a patient monitoring system 1 according to the present invention. This embodiment represents a variation of the setup shown in Fig. 1A in order to measure parasternal EMG. For this purpose the electrode pair 24, 25 is placed at the positions that maximize the contribution of parasternal intercostal muscles to the acquired signals. According to this embodiment, the monitoring device 10 may be implemented with five signal interfaces as in a typical 5-lead ECG application.

The patient monitoring system 1 may optionally further comprise an output device 30 configured to issue the determined ECG information and/or the determined EP information, and optionally the obtained electrode signal. The output device 30 may generally be any means that outputs the determined information in visual and/or audible form, e.g. in text form, as image or diagram, as sound or spoken words, etc. For instance, the output device 30 may be a display, a loudspeaker, a touchscreen, a computer monitor, the screen of a smartphone or tablet, a screen of the patient monitoring device 10, etc.

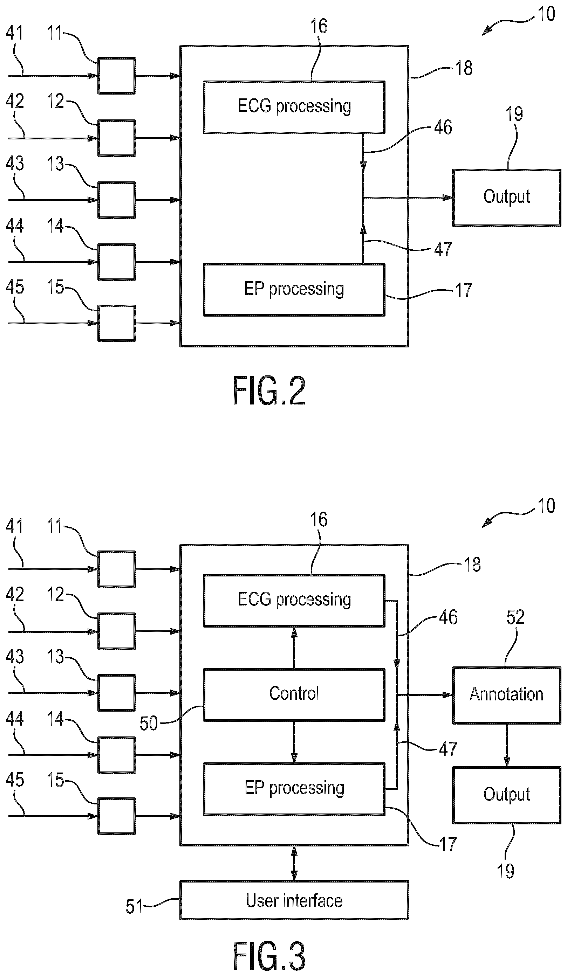

Fig. 2 shows a schematic diagram of an embodiment of a patient monitoring device 10 according to the present invention. The patient monitoring device 10 comprises a plurality (in this example five) of signal interfaces 11-15 configured to obtain a plurality of electrode signals 41-45 acquired by electrodes 21-25 placed at different positions at a patient's body as shown in Fig. 1B. The patient monitoring device 10 further comprises ECG signal processing circuitry 16 configured to apply ECG signal processing to a first set of electrode signals 41-43 (in this example three electrode signals acquired by the ECG electrodes 21-23) obtained at a first set of signal interfaces 11-13 (in this example three signal interfaces) of the plurality of signal interfaces to obtain ECG information 46, and EP signal processing circuitry 17 configured to apply EP signal processing different from the ECG signal processing to a second set of electrode signals 44 and 45 (in this example dual electrode signals acquired by the EMG electrodes 24 and 25) obtained at a second set of signal interfaces 14 and 15 (in this example two signal interfaces) of the plurality of signal interfaces to obtain EP information 47.

The ECG signal processing circuitry 16 is configured, in a first operation mode, to process the first set of electrode signals 41-43 simultaneously to the processing of the EP signal processing circuitry 16 of the second set of electrode signals 44 and 45, and, in a second operation mode, to apply ECG signal processing to the first and second sets of electrode signals 41-45.

The signal interfaces 11-15 may be configured as plugs or sockets for connecting cables of the respective electrodes. They are preferably configured as universal interfaces, i.e., it is preferably not predetermined and fixed to which of the interfaces an ECG electrode needs to be connected and to which of the interfaces an EP (e.g. EMG) electrode needs to be connected. Any electrode 21-25 can generally be connected to any of the signal interfaces. Consequently, the first and second sets of signal interfaces 11-15 are preferably not predetermined and fixed, but can be flexibly selected, i.e., there are no predetermined and fixed connections between some of the signal interfaces to only the ECG signal processing circuitry 16 or the EP signal processing circuitry 17.

The ECG signal processing circuitry 16 and the EP signal processing circuitry 17 may generally be any kind of means configured to process the electrode signals and determine the ECG information (e.g. heart rate, inter-beat intervals, QRS complex, etc.) and EP information (e.g. respiratory information like respiration rate, respiratory effort, etc.) there from. They may be implemented in software and/or hardware, e.g. as a programmed processor or computer. In an embodiment, as shown in Fig. 2, they may be part of a common processing device 18.

The patient monitoring device 10 may optionally further comprise an output unit 19 configured to output the determined ECG information and/or EP information, and optionally the obtained electrode signal. The output unit 19 may generally be any interface that provides the determined information and/or the obtained electrode signals, e.g. transmits them to another device or provides them for retrieval by another device (e.g. a smartphone, computer, tablet, server, database, storage, central monitoring station, etc.). It may thus generally be any (wired or wireless) communication or data interface.

In an embodiment a primary output unit 19 (e.g. a display) may be in the same enclosure as the common processing device 18 and an optional second output unit (e.g. a separate display) may be provided in a separate enclosure.

Thus, in an embodiment, the present invention presents a patient monitoring system and device using ECG signal acquisition means for standard ECG monitoring or impedance respiration monitoring by applying ECG signal processing, and using other ECG signal acquisition means of such a patient monitor, which are currently not used for the ECG or impedance respiration monitoring of a patient, to sense and process additional electrophysiological signals from the same patient in order to determine additional physiological information that is different from ECG or impedance respiration monitoring by processing those signals differently than it is done for ECG monitoring or impedance respiration monitoring.

Fig. 3 shows a schematic diagram of another embodiment of a patient monitoring device 10 according to the present invention. According to this embodiment the patient monitoring device 10 may optionally further comprise one or more of a control unit 50, a user interface 51, and an annotation unit 52.

The control unit 50, which may be implemented in soft- and/or hardware, e.g. as controller or programmed processor, switches the ECG signal processing circuitry 16 and/or the EP signal processing 17 between the first operation mode and the second operation mode, preferably in an automated fashion. For this switching, various information may be used, e.g. one or more of:
- user input, e.g. an input of the user indicating that one or more EP electrodes have been connected to one or more signal interfaces 11-15 or an input of the user indicating which kind of accessory is being used;
- accessory information conveyed by the measurement accessories (i.e. information on the measurement accessories) used for acquiring and/or transporting the electrode signals, e.g. information indicating the kind of signal(s) (ECG vs. EP) connected to the signal interface(s) 11-15, which information may be detected by the patient monitoring device 10 e.g. from a QR code, a color code, data storage means, etc. of the accessory; and
- an evaluation of one or more parameters of obtained electrode signals, e.g. the signal intensity, the signal curve (e.g. showing a typical ECG signal curve including a QRS complex or not), signal relationship (e.g. do signal combinations result in known ECG vector (ECG lead) relationships), etc.

The user interface 51, which may be implemented as display, touchscreen, etc., notifies the user of the operation mode and/or any mismatch regarding the actual operation mode based on the accessory information conveyed by the measurement accessory used for acquiring and/or transporting the electrode signals and/or based on an evaluation of one or more parameters of obtained electrode signals. Hence, it may be automatically detected if the actual operation mode is correct, in particular if it corresponds to the used accessory and/or if the acquired electrode signal indicates that the actual operation mode is correct or not. For instance, if the actual operation mode is the second (ECG) processing mode, but one or more electrode signals are clearly no ECG signals according to standardized electrode placements, but e.g. EMG signals, a corresponding notification such as a failure notice may be issued to the user of the patient monitoring device so that the user can check the situation and take appropriate action.

The annotation unit 52, which may be implemented in soft- and/or hardware, e.g. as controller or programmed processor, annotates the obtained ECG information and/or the obtained EP information based on one or more of the operation mode, accessory information conveyed by the measurement accessory used for acquiring and/or transporting the electrode signals, and an evaluation of one or more parameters of obtained electrode signals. Thus, rather than manual annotation, an automated annotation can be realized.

According to an embodiment, the sensed additional EP signals are referenced to the same reference electrode that is also used for the standard ECG monitoring, or the sensed additional EP signals are referenced to a non-reference electrode of the standard ECG monitoring, or the sensed additional EP signals are referenced to a separate reference electrode by using one of the other ECG signal acquisition means, which are currently not used for the ECG or impedance respiration monitoring of a patient.

Further refinements regarding the use of the different embodiments of the present invention are related to which additional EP signals are preferably addressed by the invention. According to such an embodiment, the additional physiological information based on the sensed additional EP signals is an EMG based signal, which may be related to respiratory effort, or where the EMG based signal is used to calculate neural respiratory drive (NRD) or a corresponding neural respiratory drive index (NRDI), or the EMG based signal is used to determine the current phase of the breathing cycle, or the EMG based signal is used to generate a trigger signal to indicate a certain point or event in the breathing cycle.

In a preferred embodiment, both the ECG signal acquisition and the acquisition of the additional EP signals (e.g. EMG) are done in a unified way and time wise closely synchronized, which enables further benefits. Furthermore, the standard ECG processing already makes complex and well-established analysis of the ECG related signals. This includes, for example, the detection of pacemaker spikes (pacer spikes) and their removal from the signal based on established models. This available and sophisticated information can now also be used for the benefit of the processing of the additional EP signals to enable or improve the determination of the additional physiological information. This may only be possible if there is a clear relationship and synchrony between the standard ECG signal acquisition and the acquisition of the additional EP signals. Furthermore, the standard ECG processing already performs signal quality assessments based on the known properties of ECG. Having time synchronous access to this information that relates to one and the same data acquisition device can be used beneficially also for the signal processing and signal integrity assessment of the additional EP signals. These benefits can be achieved by taking the synchronously available ECG wave samples information itself or the results from the ECG signal processing into account (together "information from the ECG signal processing") or to use the relationship between the ECG signals and the additional EP signals to obtain additional information.

These further benefits may be divided in three categories:
a) an optimized removal of cardiac crosstalk in the additional EP signals;
b) an optimized handling of external noise components in the additional EP signals; and
c) improved usability features to identify the additional EP signals from the correct electrodes and distinguish them clearly from the ECG signals.

Embodiments of the present invention that relate to category a) (the optimized removal of cardiac crosstalk in the EMG/EP signals) process those signals including attenuation or removal of cardiac crosstalk in the sensed EP signals by using information from the ECG signal processing, wherein the use of information from the ECG signal processing relates to the determination of the exact timing of the QRS complex, or wherein the use of information from the ECG signal processing relates to the determination of the amplitude of the QRS complex, or wherein the use of information from the ECG signal processing relates to the shape of the QRS complex, or wherein the use of information from the ECG signal processing relates to the detection of pacer spikes in the signal, or wherein the use of information from the ECG signal processing relates to the removal of pacer spikes from the signal.

Embodiments of the present invention that relate to category b) (the optimized handling of external noise components in the additional EP signals) process those signals including external noise detection or attenuation in the sensed EP signals by using information from the ECG signal processing, wherein the use of information from the ECG signal processing relates to determination of common mode noise visible across the various ECG leads, or wherein the use of information from the ECG signal processing relates to the determination of instances in time where noise is present at levels that prevent a reliable evaluation of the sensed EP signals, or wherein the use of information from the ECG signal processing relates to obtain a virtual reference electrode signal for the sensed EP signals by using also at least one of the ECG electrode signals.

Establishing a "dual use" of the existing ECG signal acquisition device require determining in which mode (also called "operation mode") the system actually shall operate in order to apply the correct processing to the different signal, either standard ECG / impedance respiration processing or the different processing that is needed for the additional physiological information. The mode of operation can be selected i) by the user providing input on a user interface (51 in Fig. 3) of the system 1, and/or ii) based on accessory information of specific accessories that are labelled accordingly and that convey information of the associated mode of operation, and/or iii) automatically. All these concepts may be implemented in embodiments of the present invention as described in more detail below.

The patient monitoring system may be equipped with a selectable "electrode use mode" to determine, which of the sensed signals shall be processed as standard ECG signals and which signals shall be used to determine the additional physiological information. As shown above, in an embodiment a user interface may be provided that allows the user to select the electrode use mode. **In** another embodiment, the patient monitoring system selects the electrode use mode based on information conveyed by a measurement accessory that is used to connect the electrode to the ECG signal acquisition means of the patient monitor, wherein the patient monitoring system informs the user about the electrode use mode and/or annotates the data with the electrode use mode.

Even when it is clear, which electrodes shall be used for standard ECG monitoring and which electrodes shall be used for the determination of the additional physiological information, there can be still the question which electrode placement shall be used for the determination of the additional physiological information. For instance, for the use case of respiratory effort there at two different approaches that are commonly used. One approach is to sense the diaphragm muscle as this is a large muscle that generates large EMG signals. Another approach is to use a parasternal electrode placement that senses a different and smaller respiratory muscle, but that placement has been shown to be less impacted by artefacts from other muscles. The information about the actual electrode placement for the determination of the additional physiological information may be important to be included in the patient record, and it may also be beneficial to adjust the algorithmic processing according to the electrode placement. The system may even be used for various different additional physiological information types that require completely different processing and/or algorithms.

To enable these optional additional flexibilities an analysis mode for the additional physiological information may be provided in an embodiment. The patient monitoring system can further be equipped with a selectable "analysis mode" to inform the algorithmic processing unit in which way the additional EP signals shall be processed to obtain (determine) the additional physiological information. For instance, the user interface may allow the user to select the analysis mode, or the patient monitoring system selects the analysis mode based on information conveyed by a measurement accessory that is used to connect the electrode to the ECG signal acquisition means of the patient monitor, wherein the patient monitoring system informs the user about the analysis mode and/or annotates the data with the analysis mode.

According to category c) above (improved usability features), the benefit of an embodiment of the invention to have the ECG signal acquisition and the acquisition of the additional EP signals done in a unified way and time wise closely synchronized allows to use the relationship between the ECG signals and the additional EP signals to derive additional insights of the location of the electrodes, which enable improved usability features to identify the additional EP signals from the correct electrodes and distinguish them clearly from the ECG signals.

ECG signal processing has been done for decades and is based on the principle of evaluating different vectors of the same signal source, the heart. These standardized vectors (leads) are the signal differences between certain pre-defined electrode positions on the body to look at the heart from different angles. There are even methods to synthesize / reconstruct standardized leads from specialized electrode positions to reduce the number of needed electrodes to still get the different views of the heart. Due to the propagation of the ECG electrical signals in the body each electrode position has a specific property (phase, amplitude, shape) in relation to the other electrode placements. This fact can be used to approximate the location of an electrode when its representation of the ECG signal is compared to the ECG signals that are available from the pre-defined standardized ECG electrode positions. This is particularly possible in an embodiment, which may require that the signals from standardized ECG electrode positions are acquired in a unified way and time wise closely synchronized with the additional EP signals coming from the additional electrodes.

Further embodiments of the present invention relate to improved usability features. The patient monitoring system may further perform an electrode placement analysis of the sensed signals from the ECG signal acquisition means to compare the signals of those ECG signal acquisition means that are intended for dual use with the signals from the at least 3-lead standard ECG signals in such a way to determine whether the signals for the dual use leads are likely to come from electrodes that are placed on the patient's body according to standardized 5-lead, 6-lead, 12-lead or any other standardized lead configurations or not. A notification may be provided to the user of the patient monitoring system based on the electrode placement analysis in case the configured electrode use mode does not match the sensed signals. Further, a notification may be provided to the user of the patient monitoring system based on the electrode placement analysis in case the signals sensed from the connected electrodes do not match the electrode use mode conveyed by a connected accessory. Still further, the electrode use mode may be selected automatically between standard ECG signal analysis and analysis of additional EP signals based on the electrode placement analysis. The user may be informed about the performed automatic electrode use mode selection and/or the data may be annotated with the performed automatic electrode use mode selection.

This concept can also be applied to those additional EP signals that shall be used to obtain (determine) additional physiological information to evaluate the correct electrode placement for these additional EP signals or to determine the source of the signal to tune the analysis accordingly, e.g., to detect whether the electrodes intended for respiratory effort measurements are placed to sense the diaphragm muscle or are placed in a parasternal setup. The patient monitoring system may thus further perform an electrode placement analysis of the sensed signals from those ECG signal acquisition means that are selected to be used for acquisition of additional EP signals to obtain (determine) additional physiological information. A notification may be provided to the user of the patient monitoring system based on the electrode placement analysis in case the selected analysis mode for the additional physiological information does not match the sensed signals. Further, a notification may be provided to the user of the patient monitoring system based on the electrode placement analysis in case the signals sensed from the connected electrodes do not match the selected analysis mode for the additional physiological information as conveyed by a connected accessory. Still further, the analysis mode for the additional physiological information may be selected automatically based on the electrode placement analysis.

The user may be informed about the performed automatic electrode use mode selection and/or the data may be annotated with the performed automatic electrode use mode selection.

As described above one aspect of the present invention is the use of specialized accessories to support the users in the workflow. The specialized accessories may include trunk cables and/or lead-sets that are optimized for usability in regard to cable length, or color coding, or labelling. For instance, the cable length may be adjusted to a specific electrode placement for a given electrode use mode, or the cable length may be adjusted to a specific electrode placement for a given analysis mode, or the cable length may be adjusted to the patient category and the preferred electrode placement for this patient category to obtain the additional physiological information. For instance, the cable length for neonates may be adjusted to obtain respiratory effort from electrodes placed for diaphragm EMG signals, while the cable length for adults may be adjusted to obtain respiratory effort from EMG signals with parasternal electrode placement.

Further, the specialized accessories may include trunk cables and/or lead-sets that have means to convey additional information to the patient monitoring system about the use of the signals sensed by the signal acquisition means The additional information of such specialized accessories can automatically enable certain system features as described above, which reduce the likelihood of undetected use errors and thus make the system more usable and safer. The additional information may be related to the electrode use mode and/or the analysis mode. As described above this information may cover various aspects to inform the patient monitoring system about the intended use of the accessory in regard to various aspects like the overall operation mode, the intended patient population, or the specific EP signal processing function. This additional information may also be configured to convey the intended purpose of an individual lead that is connected to a signal interface of the patient monitoring device regarding the intended function of such a lead or the intended body location of the corresponding electrode.

The additional information of such specialized accessories might also provide to the system detailed accessory specific properties in regard to signal propagation properties that can be used to improve the process to synchronize the electrode signals that come from the various locations on the body.

In order to optimize the synchronization of the various signals and/or to optimize the common mode noise rejection, it is beneficial to understand the cable design and its impact to the signal propagation on the wires and/or the influence of the cable design to the overall signal propagation and/or signal forming when the cable is connected to the signal interface (together referred to as "signal propagation properties"). This applies to both the general EP processing and the specific ECG processing.

The cable design, the used materials and actual material thickness influence these signal propagation properties. As any of these parameters might change from time to time, e.g., new regulations on substances might trigger material changes or changes are introduced by design changes or due to changes in the manufacturing process of the cable/lead-set. Even during the production of the same design and the same materials, variations in the signal propagation properties can occur, which can be determined for each manufactured cable/lead-set or per production batch.

To protect the signal interfaces of the patient monitoring device the cable/lead-set may contain voltage limiting means that might have non-linear impact on the signal propagation and in care settings where an electrosurgical unit (ESU) is used specific ESU protection means may be integrated into the cable/lead-set, which also impact the signal propagation properties.

One aspect of the invention is to consider these variabilities and to convey to the patient monitoring device one or more of the following cable/lead-set properties that impact the signal propagation: Length of signal wire, length of cable, capacity of signal wire to shield (expressed as capacity per length), capacity of signal wire to other signal wires (expressed as capacity per length), total capacity of signal wire to shield, total capacity of signal wire to other signal wires, inductance of signal wire (expressed as inductance per length), total inductance of signal wire, filter characteristic of signal wire when connected to a known impedance of the signal interface, ESU related protection means yes/no, impedance of ESU related protection means, signal amplitude protection means yes/no, characteristics of signal amplitude protection means, wire class identifier with properties known by the patient monitoring device, cable class identifier with properties known by the patient monitoring device.

In a setup where a multi-wire cable (also known as trunk cable) is used together with detachable lead-sets, conveying both the signal propagation properties of the lead-set and the signal propagation properties of the (trunk) cable might be necessary to achieve the full potential of the optimization. In such a case both accessories, the (trunk) cable as well as the lead-set, convey their own properties to the patient monitoring device.

In another embodiment only one element, preferably the one with the larger impact on the variability of the signal propagation properties, conveys its properties to the patient monitoring device.

The means to convey the additional information to the patient monitoring system may be digital means like NFC chips, memories, micro controller, or may be analog means like resistors, capacitors, inductors, light reflection, light absorption, or may be passive coding means, like bar codes, QR codes, alpha-numeric codes, which can be read by the patient monitoring device or entered by the user via the user interface of the patient monitoring device.

In an exemplary implementation, for an accessory for the example shown in Fig. 1A, the necessary information may be coded as follows:
- Operating mode = ECG + diaphragm EMG
- EMG processing function = diaphragm
- Purpose of Lead 4 (24 in Fig. 1A) is diaphragm EMG
- Body location of Lead 4 (24 in Fig. 1A) is diaphragm
- Patient category is neonatal.

In another exemplary implementation, for an accessory for the example shown in Fig. 1B, the necessary information may be coded as follows:
- Operating mode = ECG + parasternal EMG
- EMG processing function = parasternal
- Purpose of Lead 4 (24 in Fig. 1B) and/or Lead 5 (25 in Fig. 1B) is parasternal EMG
- Body location of Lead 4 (24 in Fig. 1B) is parasternal left and/or body location of Lead 5 (25 in Fig. 1B) is parasternal right
- Patient category is adult or pediatric

In summary, having access to high quality respiratory effort / respiratory neural drive information of a patient in a cost effective and workflow optimized manner as proposed by the present invention will enable continuous monitoring of these parameters. These parameters are useful not only for ventilation optimization, but also to better judge the current patient's condition, disease progression and respiratory exacerbations. It also can be an important information to judge the patient's readiness for certain steps in the clinical pathway (e.g. ventilation weaning, extubation) or the readiness to be transferred to another (lower acuity) care setting.

There are different scenarios to use the present invention in patient monitoring products. For instance, an algorithm implementation for EMG based respiratory effort determination, e.g. using an analytics platform, may be applied to a conventional ECG data acquisition system of multi-measurement modules. In another scenario, an EMG based respiratory effort software addition may be applied to an existing ECG data acquisition system of multi-measurement modules together with an existing ECG measurement function. Still further, in another scenario, an EMG based respiratory effort software addition may be made using a new EMG optimized data acquisition system, i.e. an improved ECG measurement function of a multi-measurement module and potentially new specific accessories that enable a user-friendly use of such EMG based respiratory monitoring. The present invention may further be used to monitor COPD patients or other acute care patients. This approach may use a parasternal electrode placement together with a dedicated EMG operating mode to measure and analyze the signals, potentially even without simultaneously acquiring ECG data.

The present invention enables new functionality for the portfolio of hospital patient monitoring devices that are used in the ED, OR, ICU, NICU and step-down units. Furthermore, emergency care application (e.g. defibrillation monitors) as well as applications for COPD patients, in-hospital as well as outside the hospital may benefit from the present invention, i.e. when ECG monitoring may also be necessary. The present invention can also be applied in lower acuity settings whenever patients with risk for respiratory exacerbations shall be monitored.

Preferred embodiments of the presented measurement system, device and method provide one or more of the following advantages:
- an optimized removal of cardiac crosstalk in the EMG (or EP) signals;
- an optimized handling of external noise in the EMG (or EP) signals;
- improved usability features to identify the EMG (or EP) signals from the correct electrodes and distinguish them clearly from the ECG signals; and
- improved trunk cables and/or lead-sets to identify the dual use, i.e. the simultaneous use for ECG and EMG (or EP).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Patient monitoring device (10) comprising:
- a plurality of signal interfaces (11-15) configured to obtain a plurality of electrode signals (41-45) acquired by electrodes (21-25) placed at different positions at a patient's body;
- electrocardiographic (ECG) signal processing circuitry (16) configured to apply ECG signal processing to a first set of electrode signals obtained at a first set of signal interfaces of the plurality of signal interfaces to obtain ECG information; and
- electrophysiological (EP) signal processing circuitry (17) configured to apply EP signal processing different from the ECG signal processing to a second set of electrode signals obtained at a second set of signal interfaces of the plurality of signal interfaces to obtain EP information,
wherein,
- in a first operation mode, the ECG signal processing circuitry (16) is configured to apply ECG signal processing to the first set of electrode signals and the EP signal processing circuitry (17) is configured to apply EP signal processing to the second set of electrode signals, and,
- in a second operation mode, the ECG signal processing circuitry (16) is configured to apply ECG signal processing to the first and second sets of electrode signals.

2. Patient monitoring device according to claim 1,
wherein the EP signal processing circuitry (17) is configured to use, in the first operation mode, one or more of the first set of electrode signals and/or a processing result of the simultaneous processing of the ECG signal processing circuitry of the first set of electrode signals in the processing of the second set of electrode signals.

3. Patient monitoring device according to claim 2,
wherein the EP signal processing circuitry (17) is configured to use, in the first operation mode, one or more of the first set of electrode signals and/or a processing result of the simultaneous processing of the ECG signal processing circuitry of the first set of electrode signals to reduce or remove cardiac crosstalk in the second set of electrode signals, in particular by use of one or more of:
- timing and/or amplitude and/or shape of a QRS complex in one or more of the first set of electrode signals, and
- presence and/or removal of pacer spikes in one or more of the first set of electrode signals.

4. Patient monitoring device according to claim 2 or 3,
wherein the EP signal processing circuitry (17) is configured to use, in the first operation mode, one or more of the first set of electrode signals and/or a processing result of the simultaneous processing of the ECG signal processing circuitry of the first set of electrode signals to reduce or remove external noise components in the second set of electrode signals, in particular by use of one or more of:
- common mode noise visible in one or more electrode signals of the first set,
- instances of time in which external noise above a threshold is present one or more electrode signals of the first set,
- a virtual reference electrode signal for the electrode signal of the second set from at least one electrode signal of the first set.

5. Patient monitoring device according to any one of the preceding claims,
wherein the ECG signal processing circuitry (16) and/or the EP signal processing circuitry (17) are configured to identify if an electrode signal obtained at a signal interface is an ECG signal or another EP signal and to switch the ECG signal processing circuitry into the first or second operation mode depending on the result of said identification, in particular to switch the ECG signal processing circuitry into the second operation mode if no EP signal different from an ECG signal has been identified at any signal interface.

6. Patient monitoring device according to any one of the preceding claims,
wherein the EP signal processing circuitry (17) is configured to reference the second set of electrode signals to one of:
- a reference signal among the first set of electrode signals,
- a non-reference signal among the first set of electrode signals,
- another electrode signal of the second set of electrode signals, and
- a combination of multiple electrode signals obtained at any signal interface.

7. Patient monitoring device according to any one of the preceding claims,
further comprising a control unit (50) configured to switch the ECG signal processing circuitry and/or the EP signal processing between the first operation mode and the second operation mode based on user input and/or based on accessory information conveyed by the measurement accessory used for acquiring and/or transporting the electrode signals and/or based on an evaluation of one or more parameters of obtained electrode signals.

8. Patient monitoring device according to any one of the preceding claims,
wherein the EP signal processing (17) is configured to apply or adapt EP signal processing based on user input and/or based on accessory information conveyed by the measurement accessory used for acquiring and/or transporting the electrode signals and/or based on an evaluation of one or more parameters of obtained electrode signals.

9. Patient monitoring device according to any one of the preceding claims,
further comprising:
- a user interface (51) configured to notify the user of the operation mode and/or any mismatch regarding the actual operation mode based on accessory information conveyed by the measurement accessory used for acquiring and/or transporting the electrode signals and/or based on an evaluation of one or more parameters of obtained electrode signals, and/or
- an annotation unit (52) configured to annotate the obtained ECG information and/or the obtained EP information based on one or more of the operation mode, accessory information conveyed by the measurement accessory used for acquiring and/or transporting the electrode signals, and an evaluation of one or more parameters of obtained electrode signals.

10. Patient monitoring device according to any one of the preceding claims,
wherein the first set of signal interfaces (11-13) comprises at least three signal interfaces and the second set (14, 15) of signal interfaces comprises at least one signal interface, and/or wherein the first and second sets of signal interfaces are no predetermined signal interfaces.

11. Patient monitoring device according to any one of the preceding claims,
wherein ECG signal processing circuitry (16) is configured for ECG based monitoring and/or to obtain ECG based cardiac information and/or for impedance respiration monitoring and/or to obtain impedance respiratory information; and/or
wherein the EP signal processing circuitry (17) is configured to apply electromyographic (EMG) signal processing, in particular for EMG based respiratory monitoring and/or to obtain one or more of respiratory phase, respiratory trigger signal, respiration rate, breathing effort, and breathing wave.

12. Measurement accessory, in particular measurement cable or lead-set, configured to acquire and/or transport an electrode signal acquired by an electrode of a plurality of electrodes configured to be placed at different positions at a patient's body and to acquire electrode signals, and to convey one or more of its signal propagation properties, its intended operation mode, its intended purpose, an intended electrode position on the patient's body, an intended patient population, and an intended EP signal processing function to a patient monitoring device according to any one of claims 1 to 11.

13. Patient monitoring system (1) comprising:
- measurement accessory (20), in particular a measurement accessory according to claims 14 to 20, used for acquiring and/or transporting the electrode signals (41-45) acquired by a plurality of electrodes (21-25) configured to be placed at different positions at a patient's body and to acquire electrode signals; and
- a patient monitoring device (10) according to any one of the preceding claims.

14. Patient monitoring method comprising:
- obtaining a plurality of electrode signals (41-45) acquired by electrodes (21-25) placed at different positions at a patient's body;
- applying, by electrocardiographic (ECG) signal processing circuitry (16), ECG signal processing to a first set of electrode signals obtained at a first set of signal interfaces of the plurality of signal interfaces to obtain ECG information; and
- applying, by electrophysiological (EP) signal processing circuitry (17), EP signal processing different from the ECG signal processing to a second set of electrode signals obtained at a second set of signal interfaces of the plurality of signal interfaces to obtain EP information,
wherein,
- in a first operation mode, the ECG signal processing circuitry (16) is configured to apply ECG signal processing to the first set of electrode signals and the EP signal processing circuitry (17) is configured to apply EP signal processing to the second set of electrode signals, and,
- in a second operation mode, the ECG signal processing circuitry (16) is configured to apply ECG signal processing to the first and second sets of electrode signals.

15. Computer program comprising program code means for causing a computer to carry out the steps of the method as claimed in claim 14 when said computer program is carried out on the computer.
